# Europäisches Patentamt
## European Patent Office
## Office européen des brevets

(19)

(11) Publication number: **0 095 940**
**B1**

(12) # EUROPEAN PATENT SPECIFICATION

(45) Date of publication of patent specification: **25.05.88**

(51) Int. Cl.⁴: **A 61 F 2/06**

(21) Application number: **83303149.5**

(22) Date of filing: **01.06.83**

(54) **Improvements in synthetic vascular grafts and methods of manufacturing such grafts.**

(30) Priority: **02.06.82 GB 8216066**
**02.06.82 GB 8216067**
**16.06.82 GB 8217487**

(43) Date of publication of application:
**07.12.83 Bulletin 83/49**

(45) Publication of the grant of the patent:
**25.05.88 Bulletin 88/21**

(84) Designated Contracting States:
**AT BE DE FR IT NL SE**

(56) References cited:
**EP-A-0 005 035**
**EP-A-0 009 941**
**FR-A-2 416 686**
**GB-A-1 104 680**

(73) Proprietor: **ETHICON INC.**
**U.S. Route 22**
**Somerville New Jersey 08876 (US)**
(73) Proprietor: **THE UNIVERSITY OF LIVERPOOL**
**Mount Pleasant PO BOX 147**
**Liverpool L69 3BX (GB)**

(72) Inventor: **How, Thien Voon**
**11, Watergate Way**
**Woolton Liverpool L25 8TP (GB)**
Inventor: **Clarke, Roy Malcolm**
**29 Holly Lane**
**Marston Green Birmingham B37 7AP (GB)**

(74) Representative: **Jones, Alan John et al**
**CARPMAELS & RANSFORD 43 Bloomsbury**
**Square**
**London, WC1A 2RA (GB)**

EP 0 095 940 B1

Courier Press, Leamington Spa, England.

**Description**

The invention relates to synthetic vascular grafts and their manufacture.

Various proposals have been made for manufacturing synthetic vascular grafts; for example knitted and woven grafts of Dacron* and Teflon* have been made, and extracted or dipped grafts of various polymers have also been made. A further proposal has been made to make synthetic vascular grafts by an electrostatic spinning process, for example, as set out in a paper by Annis et all in 1978 (trans. Am. Soc. Intern. Organs). In such a process, a fibre forming organic polymeric material, such as polyurethane, in solution is discharged from one or more traversing nozzles towards an electrostatically charged mandrel. Fibres of the polymer material are drawn to and collected on the mandrel to produce a fibrous tube. The microstructure of the fibrous material produced during electrostatic spinning is also described in the Annis et al paper.

Natural arteries are in general anisotropic, and the degree of anisotropy and the elastic moduli of the arteries increase with the distance from the heart, with the exception of coronary arteries. The aorta is, however, approximately isotropic. Studies on arterial grafts have hitherto concentrated on clinical and pathological considerations; it is the aim of this invention to be able to produce synthetic vascular grafts having desired anisotropic properties.

It is the aim of this invention that the synthetic vascular grafts have properties similar to those of the natural artery the graft is intended to replace.

Variations in alignment of fibres in an electrostatic spinning process between circumferential and longitudinal deposition have been described in EP—A—0 009941, such variations having been produced by changing the field in the region of the mandrel using an auxiliary electrode. The speed of rotation of the mandrel was stated as about 300 rpm.

According to the invention there is provided a method of manufacturing a synthetic vascular graft by electrostatically spinning an organic polymeric material or a precursor thereof and collecting the spun fibres on a rotating mandrel, which method comprises the step of controlling the speed of rotation of the mandrel in a range 1500 rpm to 25000 rpm such that a desired degree of anisotropy is present in the synthetic vascular graft.

According to a further aspect of the invention there is provided apparatus for manufacturing a synthetic vascular graft by electrostatic spinning, which apparatus comprises a mandrel, means for rotating the mandrel at a speed in the range 1500 rpm to 2500 rpm, means for electrostatically charging the mandrel, means for directing organic polymeric material or a precursor thereof towards the mandrel, and means for controlling the speed of rotation of the mandrel in accordance with a method according to the invention.

The speed of rotation of the mandrel may be varied between 2000 rpm and 20000 rpm.

The speed may be kept uniform during production of a particular graft, or alternatively there may be means for varying the rotational speed of the mandrel in accordance with the traverse position of the material directing means. In such a way vascular graft having varying anisotropic properties along its length could be produced.

The mandrel may be charged at −12 kV, and may have an external diameter between 1 mm and 20 mm.

The mandrel may be tapered to form a graft having a tapering cross-section.

The invention further provides a vascular graft made by a method according to the invention.

By way of example, one embodiment of apparatus and a method according to the invention for making a vascular graft will now be described with reference to the accompanying drawings, in which:

Figure 1 is a diagrammatic illustration of electrostatic spinning apparatus;

Figure 2 is a pressure-internal diameter curve of synthetic grafts between 0.3 mm and 0.6 mm wall thickness, 4 mm internal diameter;

Figure 3 is a normalized pressure-radius plot of two grafts and two arteries;

Figure 4 shows a typical load-deformation curve for a graft sample;

Figure 5 is a plot of circumferential and longitudinal Young's moduli against stress;

Figure 6 shows load-extension curves at different mandrel rotation speeds; and

Figure 7 shows a plot of the ratio of circumferential to longitudinal Young's moduli against mandrel rotation speed.

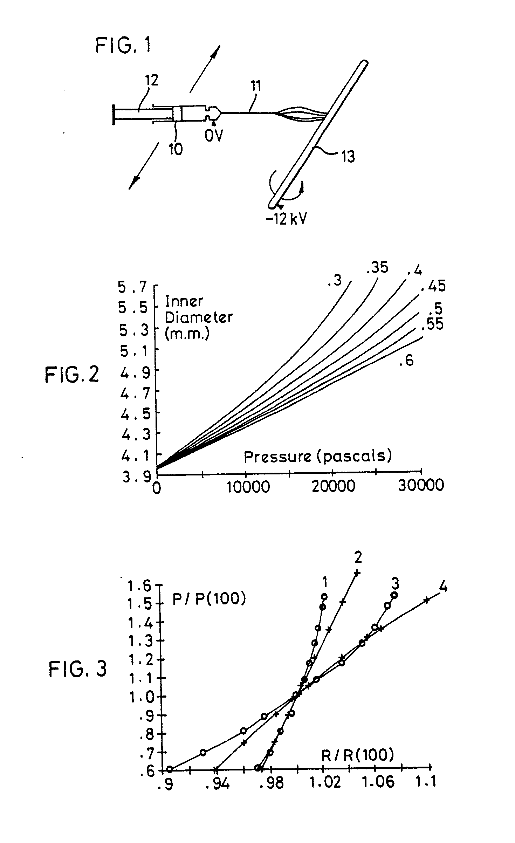

Figure 1 shows diagrammatically electrostatic spinning apparatus where a polymer solution is ejected from a syringe 10 through a nozzle 11, the nozzle 11 being earthed. To provide a constant flow of polymer solution through the nozzle 11, a syringe piston 12 is subjected to a constant hydraulic force. The particular polymer used in the following examples was a polyurethane, and the nozzle 11 was a stainless steel needle.

Fluid from the nozzle 11 is introduced in an electrostatic field surrounding a charged mandrel 13, the mandrel being charged to −12 kV. The syringe 10 is supported on a traverse mechanism which translates at a constant linear speed along the length of the mandrel. The mandrel 13 is driven by an electric motor via a drive belt and the speed of rotation of the mandrel is controlled by a thyristor controller. When a droplet of the polyurethane is introduced into the electrostatic field, the droplet elongates to form a cone or jet. From the end of the jet, fine fibres of diameter in the range 1 to 2 m are produced and are attracted onto the mandrel 13. Layers of fibres are gradually built up forming a porous and microfibrous tube.

2

Experimental analysis of the vascular grafts so produced involved the use of a mathematical model derived from experimental results.

The continuum mechanical model assumes the existence of a strain-energy function for the synthetic graft. The model assumes that the material undergoes large deformations, and is orthotropic, incompressible, homogeneous and possesses non-linear elastic properties.

The model has been used on several occasions in the past to characterise the properties of natural arteries. The model was developed by Patel et al (1972) (Patel and Vaishnav 1972 in Bergel D. H. (Ed) Cardiovascular fluid dynamics Academic Press ppl).

From the model, equations relating stresses and strains are derived, in the form of 3rd-order polynomial in strains. The constitutive equation relates stress to strain by means of seven material constanta A—G.

A series of experiments was carried out to determine the values of A—G for a 4 mm internal diameter graft. The following parameters were measured;

Unstressed dimensions:  Length
Thickness
Outside diameter

A known longitudinal stretch was applied to the graft to length L. It was then subjected to a slowly increasing intra-luminal pressure. Throughout the inflation the following parameters were continuously measured.

Pressure  P
Longitudinal force  F
Outside diameter  D

This was repeated at other lengths and for grafts of differing wall thickness.

From the data values of the constants were estimated by the method of least squares.

The strain energy density function is as follows:

$$W=Aa^2+Bab=Cb^2+DA^3+Ea^2b+Fab^2+Gb^3$$

where

$W$=strain energy density

$a$, $b$=Green St. Venant strains in the circumferential and longitudinal directions

A—G=constitutive constants

The grafts were produced with a mandrel rotation speed of 6000 r.p.m.

The values of the constants are as follows:

$(\times 10^5 Nm^{-2}\pm S.E.M.)$

| | |
|---|---|
| A=3.02±0.10 coefficient of variation= | 0.12 |
| B=2.92±0.18 | 0.21 |
| C=4.11±0.13 | 0.10 |
| D=1.70±0.24 | 0.47 |
| E=1.87±0.14 | 0.72 |
| F=2.83±0.51 | 0.59 |
| G=1.93±0.32 | 0.55 |

Although the constants D-G shows a high coefficient of variation (standard deviation/absolute mean value) their effect on the constitutive equation is less significant than the constants of A—C which control the lower-order terms since the values of a and b are less than about 0.3. The coefficients of variation are lower than those reported on any of the four occasions when the same theoretical model has been used to characterise the properties of the natural vessel.

The constitutive equation was used to predict pressure-diameter relationships for grafts having known unstressed dimensions. Predicted values were compared with measured values for all the grafts used in the experiment. The correlation was good: i.e. average value of $r^2=0.991$ with a minimum $r^2=0.983$.

Figure 2 shows a series of pressure-inside diameter relationships of 4 mm. I.D. grafts of various wall

3

thickness (0.3 to 0.6 mm in 0.05 increments), derived from the constitutive equation. All curves were calculated assuming a 6% longitudinal stretch.

Figure 3 shows the normalized pressure-radius prediction over the physiological pressure range of a 0.3 mm (curve 4) and a 0.6 mm (curve 2) wall thickness graft compared to that measured for the human carotid (curve 3) and femoral (curve 1) measured by Hayashi K. et al (J. Biomech *13*, p. 175). P (100) is a value of 100 mm Hg pressure and R (100) is the radius at that pressure.

It may be seen from the graph that the range of response of the synthetic vascular graft of the present invention which results from a change of wall thickness from 0.3 to 0.6 mm is similar to the range of properties found at different in vivo sites in the human vascular system.

The shape of the pressure-diameter curve of the sample grafts is different from the pressure-diameter curve of a natural artery. The most noticeable difference is the opposite sign of the rate of change of gradient of the curves for the synthetic and natural arteries and the more non-linear behaviour of the natural arteries. However, for a particular pressure range, selected in accordance with physiological conditions to be experienced in a particular body, a synthetic vascular graft can be made which at the limits of that selected pressure range will match the internal diameters of an in vivo natural artery. Within the selected pressure range, the synthetic graft responds similarly to the in vivo natural artery but not identically.

The constitutive equation used in relation to the 4 mm internal diameter synthetic graft is also applicable to grafts of different internal diameters, although different constants would have to be found. Furthermore, it may be possible to improve the similarity between the pressure-diameter characteristics of the synthetic graft to the characteristics of an in vivo natural artery by altering parameters in the construction of the graft. For example, the rotation speed of the mandrel has a significant effect on the shape of the load distension curve of the material, the particular graft samples of 4 mm internal diameter grafts were spun with a mandrel rotation speed of 6000 r.p.m. However, increase in the mandrel rotation speed will tend to orientate the fibres of the graft more circumferentially and such a structure is likely to be more akin to that of a natural artery. Other changes such as modifications in the electrostatic field may also alter the orientation of the fibres.

Sample preparation

Grafts of 10 mm internal diameter and wall thickness ranging from 0.3 mm to 0.7 mm were produced. In order to study the effect of changing the spinning process variables on their mechanical properties, three series of grafts were made under different manufacturing conditions. In each series one variable was altered while the others were kept unchanged. The following variables were altered:

Mandrel rotation between 1500 r.p.m. and 9000 r.p.m. (grafts M1—M6).

Traverse speed between two cm per second and 40 cm per second (grafts N1—N—4).

Concentration of spinning solution between 12 g%—16.6 g% (grafts S1—S6).

The test specimens were obtained from the cylindrical graft by opening it into a flat sheet. A die cutter was used to stamp specimens in two perpendicular directions corresponding to the circumferential and longitudinal directions of the graft. A dial gauge with a resolution of 1 m was used for thickness measurement.

Experimental procedure and results

An Instron Model TT-BM fitted with a 2000 g load cell was used for all the tests. The full scale load range could be changed electronically from 0—100 to 0—2000 g in 5 steps. The specimen was held in pneumatic grips operated at an air pressure of 500 kNm$^{-2}$. No slippage was detected between the test specimen and the grips. The specimen was therefore assumed to deform at a rate proportional to the rate of separation of the grips provided that the end effects due to clamping were minimal. All tests were carried out on dry specimens at room temperature.

Constant strain tests

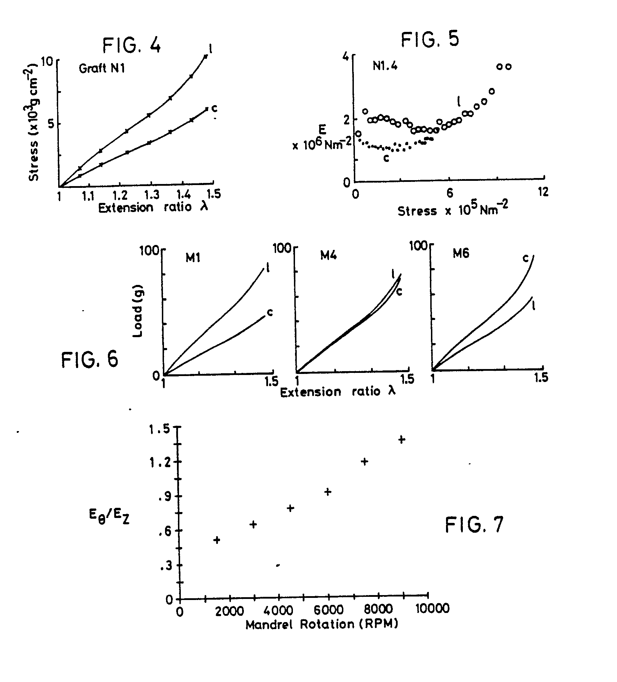

For constant strain rate tests 10 specimens were tested from each graft—5 longitudinal and 5 circumferential. The crosshead speed was set to 10 mm/min. Each specimen was preconditioned by loading and unloading it three times. The magnitude of the deformation was 50% which was the value used in the actual measurement. The specimen was then allowed to recover for 10 minutes. Because of the small amount of set (2% at 50% elongation) the gauge length was reset before actual load-extension curve was recorded. This new length was taken at the initial length $l_o$ in the subsequent analysis. The load extension curves were digitized using a Hewlett Packard 7225B plotter and HP85 desktop computer. The data points were averaged for the five specimens and were replotted in terms of Lagrangian stress, $0'$, ($0'$=load/underformed X-sectional area) and extension ratio, $\lambda$, ($\lambda$=deformed length/initial length). Figure 4 shows a typical load deformation curve. The anisotropic nature of the graft is apparent. A plot of the tensile modulus E (E=d$0'$/d$\lambda$) against tensile stress $0'$ is shown in Figure 5. The nonlinearity of the graft is clearly seen—since for a linearly elastic material E should be independent of $0'$.

Effect of electrostatic process variable on tensile properties

For each graft the average longitudinal ($E_z$) and circumferential ($E_8$) moduli were calculated using the

4

**0 095 940**

initial linear portion of the load deformation curves. In Figure 6 the load-deformation curves are replotted for 3 different mandrel rotations. The change in directional properties is clearly seen.

Table 1 shows the relationship between tensile moduli, anisotropic properties and the spinning process variables.

TABLE 1
Effect of electrostatic spinning process variables on $E_\theta$ and $E_z$

1. Variable: mandrel rotation

| Graft | Mandrel rotation (RPM) | $E_z(\times 10^6 Nm^{-2})$ | $E_\theta(\times 10^6 Nm^{-2})$ | $E_\theta/E_z$ | $E_z+E_\theta$ |
|---|---|---|---|---|---|
| M1 | 1500 | 2.10 | 1.11 | 0.53 | 3.21 |
| M2 | 3000 | 2.23 | 1.50 | 0.67 | 3.73 |
| M3 | 4000 | 1.95 | 1.57 | 0.81 | 3.52 |
| M4 · | 6000 | 1.70 | 1.64 | 0.96 | 3.34 |
| M5 | 7500 | 1.53 | 1.79 | 1.17 | 3.32 |
| M6 | 9000 | 1.33 | 1.85 | 1.39 | 3.18 |

2. Variable: traverse speed

| Graft | Traverse speed cm/s | $E_z(\times 10^6 Nm^{-2})$ | $E_\theta(\times 10^6 Nm^{-2})$ | $E_\theta/E_z$ | $E_z+E_\theta$ |
|---|---|---|---|---|---|
| N1 | 40 | 1.97 | 1.18 | 0.56 | 3.15 |
| N2 | 20 | 1.96 | 1.19 | 0.61 | 3.15 |
| N3 | 10 | 1.94 | 1.21 | 0.62 | 3.15 |
| N4 | 2 | 1.96 | 1.25 | 0.635 | 3.21 |

3. Variable: spinning solution concentration

| Graft | Solution conc. (g%) | $E_z(\times 10^6 Nm^{-2})$ | $E_\theta(\times 10^6 Nm^{-2})$ | $E_\theta/E_z$ | $E_z+E_\theta$ |
|---|---|---|---|---|---|
| S1 | 12 | 1.78 | 1.42 | 1.25 | 3.20 |
| S2 | 13 | 1.49 | 1.24 | 1.20 | 2.73 |
| S3 | 14 | 1.63 | 1.13 | 1.44 | 2.76 |
| S4 | 15 | 1.65 | 1.16 | 1.42 | 2.81 |
| S5 | 16 | 1.63 | 1.14 | 1.43 | 2.77 |
| S6 | 16,6 | 1.49 | 1.03 | 1.43 | 2.52 |

Values are mean of 5 measurements on different specimens from different parts of the graft.

Natural arteries are anisotropic in mechanical properties. With the exception of the coronary arteries, the degree of anisotropy and the elastic moduli increase with distance from the heart. Studies on the anisotropic properties of canine femoral and carotid arteries showed that at low strain the longitudinal modulus was slightly higher than the circumferential modulus, but due to the non-linearity of response of the arterial wall the circumferential modulus increased sharply at stresses greater than $5×10^5 Nm^{-2}$ whilst the longitudinal modulus changed little. Using cylindrical segments of the carotid artieries, it has been found that at an intraluminal pressure of 80 mm of mercury, circumferential Young's modulus was $8.8×10^5 Nm^{-2}$ and this increased to $1.95×10^6 Nm^{-2}$ at 140 mm Hg. Over the same pressure range the longitudinal Young's modulus increased from $8.99×10^5$ to $1.05×10^6 Nm^{-2}$. In the coronary arteries, however, the anisotropy was reversed. It has been calculated that the incremental elastic moduli of the left coronary circumflex artery and their mean values of circumferential and longitudinal Young's modulus were $7.7×10^5$ and $3.8×10^6 Nm^{-2}$ respectively.

The tensile properties of synthetic grafts with knitted and woven Dacron and Teflon have been studied. Although demonstrating a large apparent longitudinal distensibility due to the presence of circular crimps, stiffness in the circumferential direction was an order of magnitude greater than that of the natural artery.

The tensile moduli of the graft samples according to the invention listed in Table 1 were the mean values calculated between an extension ratio of about 1.0 and an extension ratio of 1.09. Circumferential Young's modulus ranged from $1.03×10^6$ to $1.85×10^6 Nm^{-2}$ and values of longitudinal Young's modulus were between $1.33×10^6$ and $2.23×10^6 Nm^{-2}$. These values of Young's modulus are slightly higher than those of the natural arteries. The grafts were less anisotropic than the carotid and coronary arteries. As can be seen from Table 1, variation in the concentration of polymer solution affected the elastic moduli. There was a tendency for the circumferential modulus to decrease with the concentration but there was no definite trend in the values of the longitudinal modulus.

As can be seen in Figures 6 and 7, it is possible, by varying the mandrel rotation speed to control the ratio of circumferential to longitudinal modulus. At low rotation, the circumferential modulus is less than the longitudinal modulus suggesting that there is a preferential alignment of fibres in the longitudinal direction. As the speed of rotation of the mandrel increases, the tube becomes isotropic and thereafter the anisotropy reverses and the circumferential modulus becomes greater than the longitudinal modulus. The traverse speed has a small influence on anisotropy but for practical purposes its effect may be ignored.

The sample grafts were made with an internal diameter of 10 mm but it will be appreciated that variation of the mandrel rotation speed will also effect anisotropy in grafts of different diameters. The smaller the graft diameter, the less effect increasing mandrel rotation speed has on increasing the ratio between circumferential and longitudinal moduli and it is proposed to test sample grafts at mandrel rotation speeds of up to between 20000 r.p.m. and 25000 r.p.m. for diameters down to 1 mm. The useful range of vascular grafts is between 1 mm and 20 mm internal diameter, and wall thickness may vary between 0.075 mm and 2 mm.

**Claims**

1. A method of manufacturing a synthetic vascular graft by electrostatically spinning an organic polymeric material or a precursor thereof and collecting the spun fibres on a rotating mandrel (13), which method comprises the step of controlling the speed of rotation of the mandrel (13) in a range 1500 rpm to 25000 rpm such that a desired degree of anisotropy is present in the synthetic vascular graft.

2. A method as claimed in Claim 1 wherein the speed of rotation of the mandrel (13) is between 2000 rpm and 20000 rpm.

3. A method as claimed in Claim 1 or Claim 2 wherein the speed of rotation of the mandrel (13) is kept uniform during production of the graft.

4. A method as claimed in Claim 1 or Claim 2 wherein the speed of rotation of the mandrel (13) is varied.

5. Apparatus for manufacturing a synthetic vascular graft by electrostatic spinning, which apparatus comprises a mandrel (13), means for rotating the mandrel (13) at a speed in the range 1500 rpm to 25000 rpm, means for electrostatically charging the mandrel (13), means (11) for directing organic polymeric material or a precursor thereof towards the mandrel (13), and means for controlling the speed of rotation of the mandrel in accordance with a method as claimed in any one of Claims 1 to 4.

6. Apparatus as claimed in Claim 5 wherein the means for rotating the mandrel rotates the mandrel at a speed in the range 2000 rpm to 20000 rpm.

7. Apparatus as claimed in Claim 5 or Claim 6 wherein the means (11) for directing organic polymeric material or a precursor thereof towards the mandrel (13) traverses the mandrel (13), the apparatus comprising means for varying the rotational speed of the mandrel (13) in accordance with the traverse position of the directing means (11).

8. Apparatus as claimed in any one of Claims 5 to 7 wherein the mandrel (13) is tapered to form a graft having a tapering cross section.

9. Apparatus as claimed in any one of Claims 5 to 8 wherein the mandrel (13) has an external diameter between 1 mm and 20 mm.

**Patentansprüche**

1. Verfahren zur Herstellung eines synthetischen Gefäßtransplantats durch elektrostatisches Spinnen

# 0 095 940

eines organischen Polymermaterials oder eines Vorläufers hievon und Auffangen der gesponnenen Fasern auf einem rotierenden Dorn (13), welches Verfahren die Stufe der Regelung der Rotationsgeschwindigkeit des Dorns (13) in einem Bereich von 1.500 bis 25.000 Umdrehungen je Minute derart umfaßt, daß in dem synthetischen Gefäßtransplantat ein gewünschter Grad an Anisotropie vorliegt.

2. Verfahren nach Anspruch 1, worin die Rotationsgeschwindigkeit des Dorns (13) zwischen 2.000 und 20.000 Umdrehungen je Minute liegt.

3. Verfahren nach Anspruch 1 oder 2, worin die Rotationsgeschwindigkeit des Dorns (13) während der Herstellung des Transplantats gleich gehalten wird.

4. Verfahren nach Anspruch 1 oder 2, worin die Rotationsgeschwindigkeit des Dorns (13) variiert wird.

5. Vorrichtung zur Herstellung eines synthetischen Gefäßtransplantats durch elektrostatisches Spinnen, welche Vorrichtung einen Dorn (13), Mittel zur Rotation des Dorns (13) bei einer Geschwindigkeit im Bereich von 1.500 bis 25.000 Umdrehungen je Minute, Mittel zum elektrostatischen Laden des Dorns (13), Mittel (11) zum Richten eines organischen Polymermaterials oder eines Vorläufers hievon gegen den Dorn (13) und Mittel zur Regelung der Rotationsgeschwindigkeit des Dorns gemäß der in einem der Ansprüche 1 bis 4 beanspruchten Methode umfaßt.

6. Vorrichtung nach Anspruch 5, worin die Mittel zur Rotation des Dorns den Dorn mit einer Geschwindigkeit im Bereich von 2.000 bis 20.000 Umdrehungen je Minute rotieren.

7. Vorrichtung nach Anspruch 5 oder 6, worin das Mittel (11) zum Richten eines organischen Polymermaterials oder eines Vorläufers hievon gegen den Dorn (13) gegenüber dem Dorn (13) verschoben wird, wobei die Vorrichtung Mittel zum Variieren der Rotationsgeschwindigkeit des Dorns (13) in Abhängigkeit von der Verschiebestellung des Richtmittels (11) umfaßt.

8. Vorrichtung nach einem der Ansprüche 5 bis 7, worin der Dorn (13) zur Ausbildung eines Transplantats mit einem sich verjüngenden Querschnitt konisch ausgebildet ist.

9. Vorrichtung nach einem der Ansprüche 5 bis 8, worin der Dorn (13) einen Außendurchmesser zwischen 1 mm und 20 mm aufweist.

## Revendications

1. Procédé de fabrication d'une greffe vasculaire de synthèse par filage électrostatique d'un matériau polymère organique ou d'un de ses précurseurs et recueil des fibres filées sur un mandrin tournant (13), ce procédé comprenant l'étape consistant à commander la vitesse de rotation du mandrin (13) dans une gamme de 1500 tours/minute à 25000 tours/minute de sorte qu'un degré souhaité d'anisotropie soit présente dans la greffe vasculaire de synthèse.

2. Procédé selon la revendication 1, dans lequel la vitesse de rotation du mandrin (13) est comprise entre 2000 tours/minute et 20000 tours/minute.

3. Procédé selon la revendication 1 ou la revendication 2, dans lequel la vitesse de rotation du mandrin (13) est maintenue uniforme pendant la fabrication de la greffe.

4. Procédé selon la revendication 1 ou la revendication 2, dans lequel la vitesse de rotation du mandrin (13) est modifiée.

5. Appareil de fabrication d'une greffe vasculaire de synthèse par filage électrostatique, cet appareil comprenant un mandrin (13), des moyens pour faire tourner le mandrin (13) à une vitesse dans la gamme de 1500 tours/minute à 25000 tours/minute, des moyens pour charger électrostatiquement le mandrin (13), des moyens (11) pour diriger un matériau polymère organique ou l'un des ses précurseurs vers le mandrin (13) et des moyens pour commander la vitesse de rotation du mandrin selon le procédé revendiqué dans l'une quelconque des revendications 1 à 4.

6. Appareil selon la revendication 5, dans lequel le moyen pour faire tourner le mandrin fait tourner le mandrin à une vitesse dans la gamme de 2000 à 20000 tours/minute.

7. Appareil selon la revendication 5 ou la revendication 6, dans lequel le moyen (11) pour diriger le matériau polymère organique ou l'un de ses précurseurs vers le mandrin (13) est déplacé latéralement en regard du mandrin (13), l'appareil comprenant des moyens pour faire varier la vitesse de rotation du mandrin (13) en accord avec la position transverse du moyen de direction (11).

8. Appareil selon l'une quelconque des revendications 5 à 7, dans lequel la mandrin (13) est conique pour former une greffe ayant une section conique.

9. Appareil selon l'une quelconque des revendications 5 à 8, dans lequel le mandrin (13) a un diamètre externe compris entre 1 mm et 20 mm.

FIG. 1

FIG. 2

FIG. 3

FIG. 4

FIG. 5

FIG. 6

FIG. 7